Europäisches Patentamt

European Patent Office

Office européen des brevets

(11) Publication number: **0 316 079**
**A1**

# EUROPEAN PATENT APPLICATION

(21) Application number: 88309789.1

(51) Int. Cl.⁴: **A61K 7/16**

(22) Date of filing: 19.10.88

(30) Priority: 20.10.87 GB 8724544

(43) Date of publication of application:
**17.05.89 Bulletin 89/20**

(84) Designated Contracting States:
**AT BE CH DE ES FR GB GR IT LI NL SE**

(71) Applicant: **UNILEVER PLC**
**Unilever House Blackfriars P.O. Box 68**
**London EC4P 4BQ(GB)**

(84) **GB**

(71) Applicant: **UNILEVER NV**
**Burgemeester s'Jacobplein 1 P.O. Box 760**
**NL-3000 DK Rotterdam(NL)**

(84) **BE CH DE ES FR GR IT LI NL SE AT**

(72) Inventor: **Ingram, Geoffrey Stewart**
**The Cairngorms 4 Stanley Avenue**
**Bebington Wirral Merseyside L63 5QF(GB)**

(74) Representative: **Doucy, Robert Henry et al**
**Unilever PLC Patents Division P.O. Box 68**
**Unilever House**
**London EC4P 4BQ(GB)**

(54) **Oral compositions.**

(57) The disclosure relates to an oral composition for inhibiting the formation of dental calculus comprising in combination sodium trimetaphosphate and a zinc salt.

EP 0 316 079 A1

## ORAL COMPOSITIONS

This invention relates to oral compositions effective to retard the formation of calculus, also called tartar.

There have recently been published proposals to formulate anti-calculus oral products on the basis of linear molecularly dehydrated polyphosphate salts. Such products are disclosed in US-A-4 627 977 (Gaffar et al), US-A-4 590.066 (Parran et al), US-A-4 515 772 (Parran et al), US-A-4 590 066 (Parran et al), US-A-4 684 518 (Parran et al), GB-A-2 182 244 (Colgate-Palmolive) and GB-A-2 188 548 (Beecham), all incorporated herein by reference. Examples of polyphosphate salts that may be present in such compositions as given in these patents are hexametaphosphates, tripolyphosphates and pyrophosphates. Prior to the publication of the above-mentioned patents it was disclosed in US-A-4 100 269 (Pader) incorporated herein by reference, that certain zinc salts, for example zinc citrate, exhibited an anti-calculus effect when incorporated in dentifrices.

US-A-4 627 977 (Gaffar et al) warns against the inclusion of zinc salts in oral products containing the polyphosphate salts because of complex formation and states that the presence of zinc salts should be avoided.

In GB-A-1 547 874 (Unilever) dentifrice compositions are described for combating dental caries comprising 0.5 to 6% by weight of sodium trimetaphosphate and a silica xerogel abrasive.

The anti-calculus oral composition of the present invention, which is preferably a toothpaste, comprises the combination of a zinc salt and sodium trimetaphosphate. Sodium trimetaphosphate, $Na_3P_3O_9$, is a cyclic condensed phosphate. More specifically the anti-calculus oral composition of the invention comprises 0.5 to 6%, preferably 1% to 5%, by weight sodium trimetaphosphate and 0.05 to 2%, preferably 0.1 to 1%, by weight of a zinc salt.

The zinc salt may be any of the sparingly water soluble salts disclosed in US-A-4 100 269. More water-soluble salts may also be used as disclosed in US-A-4 022 880 (Vinson et al), incorporated herein by reference. Other zinc salts suitable for use in oral compositions are disclosed in US-A-4 144 323 (Lamberti), US-A-4 656 031 (Lane et al) and US-A-4 160 821 (Sipos), all incorporated herein by reference. The preferred zinc salt is zinc citrate.

The oral composition of the invention may also include a source of fluoride-ions or fluorine providing compound. An amount of a fluoride source sufficient to supply from about 50ppm to about 3500ppm of fluoride ions may be used. Preferred fluorides are sodium fluoride and sodium monofluorophosphate. Other suitable sources of fluoride ions or fluorine-containing ions are disclosed in the patents referred to above.

The oral composition of the invention may also comprise a linear condensed phosphate as described in the prior patents referred to above, such as di- or tetrasodium pyrophosphate or di- or tetra-potassium pyrophosphate or a mixture thereof.

The oral composition may also comprise a synthetic anionic polymeric polycarboxylate as described in GB-A-2 182 244 (Colgate-Palmolive) or an acrylic acid polymer or copolymer as described in US-A-4 661 341 (Benedict et al). Further polycarboxylates are described in US-A-3 429 963 (Shedlovsky). All of these patents are incorporated herein by reference.

Where the oral composition of the invention is a toothpaste, the composition may also comprise an abrasive cleaning agent. While several abrasive agents suitable for toothpaste use are described in the above-mentioned prior patents, preferred abrasives are silicas, water-insoluble sodium metaphosphate and plastics materials, or mixtures thereof.

Other ingredients of oral compositions are well known to those skilled in the art. In any event, they are generally described in the aforementioned patents. Thus toothpastes will generally also comprise a humectant, binder or thickener, and surfactant.

The oral compositions of the invention may be made by standard procedures which involve blending the sodium trimetaphosphate and zinc salt with other conventional ingredients.

The following Examples illustrate the invention. Percentages are by weight.

Example 1

A toothpaste is prepared having the following composition.

2

| | % |
|---|---|
| Silica xerogel | 12.0 |
| Sorbitol syrup (70% solution) | 27.0 |
| Hydroxyethyl cellulose | 1.7 |
| Sodium lauryl sulphate | 1.5 |
| Zinc citrate trihydrate | 0.5 |
| Sodium trimetaphosphate | 3.0 |
| Sodium fluoride | 0.23 |
| Saccharin | 0.20 |
| Flavour | 1.00 |
| Water | to 100.00 |

Example 2

A toothpaste is prepared having the following composition.

| | % |
|---|---|
| Silica xerogel | 12.0 |
| Sorbitol syrup (70% solution) | 27.0 |
| Hydroxyethyl cellulose | 1.7 |
| Sodium lauryl sulphate | 1.5 |
| Zinc citrate trihydrate | 0.5 |
| Sodium trimetaphosphate | 3.0 |
| Sodium monofluorophosphate | 0.76 |
| Saccharin | 0.20 |
| Flavour | 1.00 |
| Water | to 100.00 |

Examples 3 to 7

Other formulations are made by varying the amount of zinc citrate and/or sodium trimetaphosphate in the formulation of Example 1 or Example 2 as indicated below:

| | zinc citrate(%) | sodium trimetaphosphate(%) |
|---|---|---|
| Example 3 | 1.0 | 3.0 |
| Example 4 | 0.5 | 2.0 |
| Example 5 | 1.0 | 2.0 |
| Example 6 | 0.5 | 5.0 |
| Example 7 | 1.0 | 5.0 |

The toothpastes are effective to reduce the growth of calculus.

**Claims**

1. An oral composition for inhibiting the formation of dental calculus comprising 0.5 to 6% by weight sodium trimetaphosphate and 0.05 to 2% by weight of a zinc salt.

2. An oral composition as claimed in claim 1 wherein the sodium trimetaphosphate is present in an amount of 1 to 5% by weight.

3. An oral composition as claimed in claim 1 or claim 2 comprising 0.1 to 1% by weight of a zinc salt.

4. An oral composition as claimed in any of the preceding claims wherein the zinc salt is zinc citrate.

5. An oral composition as claimed in any of the preceding claims in the form of a toothpaste.

6. An oral composition as claimed in claim 5 comprising a silica abrasive cleaning agent.

7. Method of making an oral composition s claimed in any of the preceding claims comprising blending the respective specific ingredients with other conventional ingredients.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 095 356 (H.V. MOSS et al.) <br> * Claims 1,5,9; column 1, lines 38-39 * | 1-7 | A 61 K 7/16 |
| Y | US-A-4 138 477 (M.C.S. GAFFAR) <br> * Claim 1; column 8, lines 51-68 * | 1-7 | |
| Y | BE-A- 539 808 (INDIANA UNIVERSITY) <br> * Claims 1,5,6,14 * | 1-7 | |
| A | US-A-3 699 220 (WESTSTRATE et al.) <br> * Whole document * | 1-7 | |
| A | US-A-4 309 409 (COLL- PALAGOS et al.) <br> * Column 2, line 38 - column 3, line 3; claim 1 * | 1-7 | |
| A | THE MERCK INDEX, 10th edition, 1983, page 1237, edited by M. WINDHOLZ et al., Merck & CO., Rahway, US, no. 8479: "Sodium Metaphosphate" | 1-7 | |

TECHNICAL FIELDS SEARCHED (Int. Cl.4)

A 61 K 7/00

The present search report has been drawn up for all claims

| Place of search | Date of completion of the search | Examiner |
|---|---|---|
| THE HAGUE | 23-02-1989 | GERLI P.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)